# NOUVEAU FASCICULE DE BREVET EUROPEEN

(11) **EP 1 140 250 B2**
(45) Date de publication et mention de la décision concernant l'opposition: **10.09.2014**
(45) Mention de la délivrance du brevet: 12.04.2006
(21) Numéro de dépôt: 00969757.4
(22) Date de dépôt: 02.11.2000
(51) Int. Cl.: A61M 1/16, B01F 3/08

(54) **DISPOSITIF DE PREPARATION D'UN LIQUIDE MEDICAL**
VORRICHTUNG ZUR AUFBEREITUNG EINER MEDIZINISCHEN FLÜSSIGKEIT
DEVICE FOR PREPARING A MEDICAL FLUID

(30) Priorité: 02.11.1999 IT TO990948
(43) Date de publication de la demande: 10.10.2001
(73) Titulaire: GAMBRO HOSPAL (Schweiz) AG, 4001 Basel (CH)
(72) Inventeur: BOSETTO, Antonio, 41036 Mirandolla (IT); PAOLINI, Francesco, Ganaceto, 41010 Modena (IT)
(74) Mandataire: Ponzellini, Gianmarco
(86) Numéro de dépôt international: PCT/IB2000/001588
(87) Numéro de publication internationale: WO 2001/032237

(56) Documents cités:
- EP-A1- 0 086 553
- EP-A2- 0 097 366
- WO-A-95/08299
- FR-A- 2 504 817
- US-A- 4 136 708
- US-A- 5 091 094
- US-A- 5 616 248
- D. SCHLEIPFER: 'Ein Kompendium über die technischen Grundlagen der Behandlung mit der Künstlichen Niere' AUSZUG AUS DIALYSETECHNIK vol. 4, 1988, page 156

## Description

La présente invention a objet pour objet un système de traitement extracorporel de sang.

L'invention trouve notamment une application dans le domaine du traitement de l'insuffisance rénale où elle peut être mise en oeuvre pour la préparation d'un liquide de dialyse. Dans ce cadre, l'invention est particulièrement adaptée au traitement de patients dont le milieu intérieur présente un excès de potassium.

Les reins remplissent de multiples fonctions parmi lesquelles l'élimination d'eau, l'excrétion des catabolites (ou déchets du métabolisme, tels que l'urée, la créatinine), la régulation de la concentration des électrolytes du sang (sodium, potassium, magnésium, calcium, bicarbonates, phosphates, chlorures) et la régulation de l'équilibre acido-basique du milieu intérieur qui est obtenue notamment par l'élimination des acides faibles (phosphates, acides monosodiques) et par la production de sels d'ammonium.

Chez les personnes qui ont perdu l'usage de leurs reins, ces mécanismes excréteurs et régulateurs ne jouant plus, le milieu intérieur se charge en eau et en déchets du métabolisme et présente un excès d'électrolytes (de sodium en particulier), ainsi que, en général, une acidose, le pH du plasma sanguin se déplaçant vers 7.

Pour pallier le dysfonctionnement des reins, on recourt classiquement à un traitement du sang par circulation extracorporelle dans un échangeur à membrane semi-perméable (hémodialyseur) où l'on fait circuler, d'un côté et de l'autre de la membrane, le sang du patient et un liquide de dialyse comprenant les principaux électrolytes du sang (chlorures, bicarbonates, sodium, calcium, potassium, magnésium) dans des concentrations proches de celles du sang d'un sujet sain. Par l'effet du phénomène physique appelé dialyse, les molécules migrent du liquide où leur concentration est la plus élevée vers le liquide où leur concentration est la moins élevée.

Une modification électrolytique significative chez les patients urémiques est l'augmentation de la concentration en potassium du plasma. Or l'hyperkaliémie (concentration du potassium trop élevée) est associée à des incidents liés à l'hyperpolarisation de la membrane des cellules neuromusculaires, qui peuvent avoir pour conséquence l'arythmie hypocinétique et le bloc auriculoventrulaire total. L'un des objectifs d'un traitement de dialyse est donc d'éliminer l'excès de potassium accumulé par les patients entre deux séances de traitement. Conformément au principe physique rappelé plus haut, la quantité de potassium éliminée durant le traitement dépend directement de la différence entre la concentration du potassium dans le plasma et la concentration du potassium dans le liquide de dialyse, laquelle est généralement fixée à un niveau constant, inférieur (environ 2 mEq/l) au niveau physiologique (environ 3,5 mEq/l).

Au début d'un traitement de dialyse classique, un patient hyperkaliémique (dont la concentration plasmatique en potassium peut s'élever jusqu'à 10 mEq/l) est soumis aux effets indésirables résultant de la différence importante entre la concentration en potassium de son plasma et celle du liquide de dialyse : ce gradient élevé provoque en effet un flux diffusif important de potassium au travers de la membrane de l'hémodialyseur, lequel provoque à son tour un flux important de potassium au travers de la membrane des cellules, ce qui affecte le potentiel membranaire électrique de repos et, par voie de conséquence, l'excitabilité cellulaire. Comme ce mécanisme influence aussi les cellules pace-maker cardiaques, le patient court le risque d'une arythmie cardiaque durant le traitement de dialyse. Ce phénomène est naturellement accru en cas de faiblesse cardiaque et peut conduire à une réduction du volume d'éjection affectant la circulation cardiovasculaire.

Le document US-A-5616248 expose un dispositif pour préparer un liquide de traitement selon le préambule de la revendication 1.

Un but particulier de l'invention est donc de modifier les conditions d'un traitement de dialyse classique, sans toutefois en affecter l'efficacité, de façon que les patients hyperkaliémiques ne soient plus soumis aux risques mentionnés ci-dessus.

Un but général de l'invention est de concevoir un système de traitement extracorporel de sang comprenant un dispositif pour préparer un liquide de traitement, qui puisse être utilisé pour mettre en oeuvre un traitement extracorporel de sang, au moyen duquel la concentration de deux substances ioniques puisse être ajustée séparément, notamment le sodium et le potassium (ou le calcium, ou le magnésium).

Conformément à l'invention, ce but est atteint grâce à un système de traitement extracorporel de sang comprenant un dispositif de préparation d'un liquide médical à partir d'un liquide, tel que de l'eau, et de deux solutions concentrées selon la revendication 1.

Selon une caractéristique de l'invention, on fait varier au cours du temps le débit d'injection Q1 et le débit d'injection Q2 des solutions concentrées A et B de façon que la concentration de la deuxième substance B dans la solution diluée varie au cours du temps selon un profil prédéterminé.

Selon une autre caractéristique de l'invention, le débit Q0 du liquide dans la canalisation est constant et la somme des débits d'injection Q1 + Q2 des solutions concentrées A et B est maintenue constante de sorte que la concentration de la première substance A dans la solution diluée reste sensiblement constante.

Selon encore une autre caractéristique de l'invention, on fait varier au cours du temps le débit d'injection Q1 et le débit d'injection Q2 des solutions concentrées A et B de façon que la concentration de la première substance A dans la solution diluée varie au cours du temps selon un profil prédéterminé.

Dans un mode de réalisation de l'invention, le dispositif de préparation de liquide de traitement est incorporé dans un système d'hémodialyse, la substance A est du sodium et la substance B est du potassium, du calcium, ou du magnésium. Comme la concentration en sodium dans un liquide de dialyse est très supérieure à la concentration en potassium (calcium ou magnésium), on peut régler très précisément la concentration en potassium à partir de la mesure de la conductivité du mélange se formant dans la canalisation immédiatement en aval du lieu d'injection de la première solution concentrée dans la canalisation et du mélange se formant dans la canalisation immédiatement en aval du lieu d'injection de la deuxième solution concentrée dans la canalisation (il existe une excellente corrélation entre le la conductivité d'uné solution et sa concentration en sodium).

Par ailleurs, il est sans danger d'influencer la concentration plasmatique d'un patient en potassium ou en calcium, par l'intermédiaire d'une solution de dialyse très diluée préparée et administrée au moyen d'un système muni de moyens de mesure de concentration fiables, ce qui ne serait pas le cas si l'on visait ce but au moyen d'une injection de solution plus concentrée.

L'invention a aussi pour objet un kit de solutions pour un traitement extracorporel de sang comprenant deux solutions concentrées ainsi qu'une poche à deux compartiments pour contenir chacune des solutions du kit.

D'autres caractéristiques et avantages de l'invention apparaîtront mieux à la lecture de la description qui va suivre. On se reportera aux dessins annexés sur lesquels :
La figure 1 est un schéma d'un dispositif de traitement du sang ;
La figure 2 représente une poche à deux compartiments pour contenir les deux solutions concentrées d'un kit de traitement ; et
La figure 3 est un graphe représentant plusieurs profils de variation de la concentration en potassium d'un liquide de dialyse.

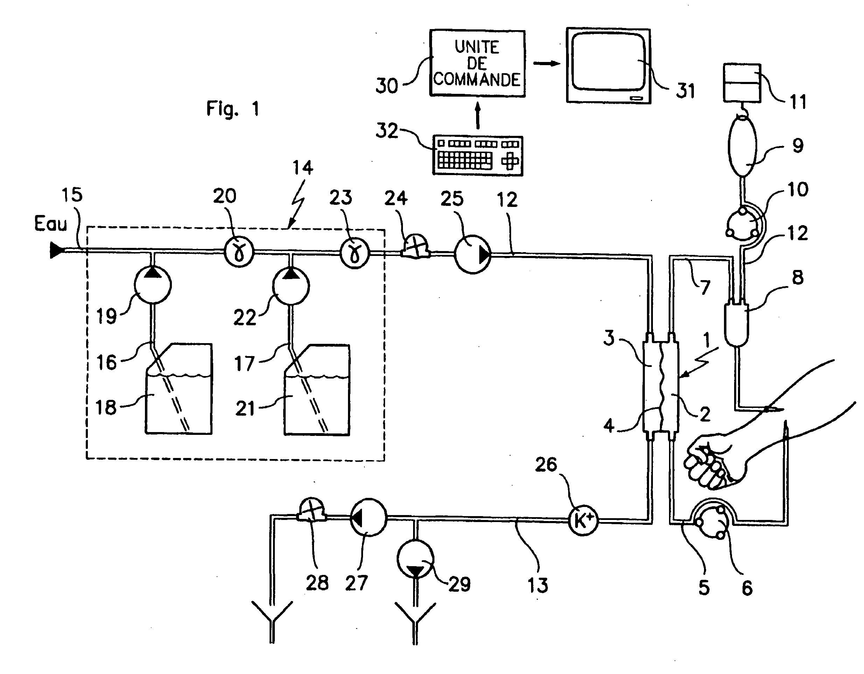

Le système d'hémodialyse représenté sur la figure 1 comprend un hémodialyseur 1 ayant deux compartiments 2, 3 séparés par une membrane semi-perméable 4. Un premier compartiment 2 a une entrée connectée à une canalisation 5 de prélèvement de sang sur laquelle est disposée une pompe de circulation 6, et une sortie connectée à une canalisation 7 de restitution de sang sur laquelle est interposé un piège à bulles 8.

Un dispositif de perfusion comprenant une pompe 10 et une balance 11 est prévu pour injecter dans le piège à bulles 8 le contenu d'une poche 9 de liquide de perfusion contenant du bicarbonate de sodium. La poche 9 est suspendue à la balance 11 et elle est reliée au piège à bulles 8 par une canalisation 12 sur laquelle est disposée la pompe de perfusion 10. La balance 11 sert à piloter la pompe 10 pour que le débit du liquide de perfusion soit égal à un débit de consigne.

Le second compartiment 3 de l'hémodialyseur 1 a une entrée connectée à une canalisation 12 d'alimentation en liquide de dialyse frais et une sortie connectée à une canalisation 13 d'évacuation de liquide usé (liquide de dialyse et ultrafiltrat).

La canalisation d'alimentation 12 relie l'hémodialyseur 1 à un dispositif de préparation de liquide de dialyse 14 comprenant une canalisation principale 15 dont l'extrémité amont est prévue pour être raccordée à une source d'eau courante. A cette canalisation principale 15 sont connectées une première et une deuxième canalisation secondaires 16, 17.

Conformément à l'invention, l'extrémité libre de la première canalisation secondaire 16 est destinée à être immergée dans un conteneur 18 pour une première solution saline concentrée contenant du chlorure de sodium, de calcium, de magnésium et de potassium. Cette première canalisation 16 est équipée d'une pompe 19 de dosage de la première solution concentrée dans le liquide de dialyse qui est pilotée en fonction de la comparaison entre 1) une première valeur de consigne de conductivité pour la solution se formant à la jonction de la canalisation principale 15 et de la première canalisation secondaire 16 et 2) la valeur de la conductivité de cette solution mesurée au moyen d'une première sonde de conductivité 20 disposée sur la canalisation principale 15 immédiatement en aval de la jonction entre la canalisation principale 15 et la première canalisation secondaire 16.

Conformément à l'invention, l'extrémité libre de la deuxième canalisation secondaire 17 est destinée à être immergée dans un conteneur 21 pour une deuxième solution saline concentrée contenant du chlorure de sodium, de calcium, de magnésium et de potassium. Cette deuxième solution contient les mêmes substances ioniques que la première solution et dans les mêmes concentrations, à l'exception du potassium dont la concentration diffère. La deuxième canalisation 17 est équipée d'une pompe 22 de dosage de la deuxième solution concentrée dans le liquide de dialyse qui est pilotée en fonction de la comparaison entre 1) une deuxième valeur de consigne de conductivité pour la solution se formant à la jonction de la canalisation principale 15 et de la deuxième canalisation secondaire 17 et 2) la valeur de la conductivité de cette solution mesurée au moyen d'une deuxième sonde de conductivité 23 disposée sur la canalisation principale 15 immédiatement en aval de la jonction entre la canalisation principale 15 et la deuxième canalisation secondaire 17.

La canalisation 12 d'alimentation en liquide de dialyse forme le prolongement de la canalisation principale 15 du dispositif 14 de préparation de liquide de dialyse. Sur cette canalisation 12 d'alimentation sont disposés, dans le sens de circulation du liquide, un premier débitmètre 24 et une première pompe de circulation 25.

L'extrémité aval de la canalisation 13 d'évacuation de liquide usé est prévue pour être reliée à l'égout. Sur cette canalisation sont disposés, dans le sens de circulation du liquide, une sonde 26 de mesure de concentration de potassium, une deuxième pompe de circulation 27, et un deuxième débitmètre 28. Une pompe d'extraction 29 est reliée à la canalisation d'évacuation 13, en amont de la deuxième pompe de circulation 27.

Le système d'hémodialyse représenté sur la figure 1 comprend également une unité de calcul et de commande 30. Cette unité est reliée à un écran 31 et à un clavier 32 par lequel l'utilisateur lui communique diverses valeurs de consigne : consignes de débit (débit de sang Qb, débit de liquide de dialyse Qd, débit de la solution de perfusion Qinf), consignes de concentration en substances ioniques dans le liquide de dialyse, consigne de durée de traitement T, consigne de perte de poids WL. Par ailleurs, l'unité de calcul et de commande 30 reçoit des information émises par les organes de mesure du système, tels que les débitmètres 24, 28, les sondes de conductivité 20, 23, la sonde 26 de mesure de concentration de potassium. Elle pilote, en fonction des instructions reçues et de modes d'opération et d'algorithmes programmés les organes moteurs du système, tels que les pompes 6, 10, 19, 22, 25, 27, 29.

Conformément à l'invention, il est possible d'ajuster indépendamment l'une de l'autre la concentration du sodium et la concentration du potassium dans le liquide de dialyse : pour un débit Q0 d'eau constant, la concentration du sodium dépend de la somme du débit Q1 de la première solution concentrée injectée par la pompe 19 dans la canalisation principale 15 et du débit Q2 de la deuxième solution concentrée injectée par la pompe 22 dans la canalisation principale 15, alors que La concentration du potassium dépend du rapport des débits Q1, Q2 de la première et de la deuxième solution concentrée. La concentration du sodium et du potassium dans le liquide de dialyse est choisie en fonction de chaque patient particulier. Elle peut est ajustée à une valeur fixe. Conformément à l'invention, pour les patients hyperkaliémiques, la concentration en potassium du liquide de dialyse est modifiée en permanence au cours de la séance de traitement selon un profil de variation prédéterminé.

### Exemple:

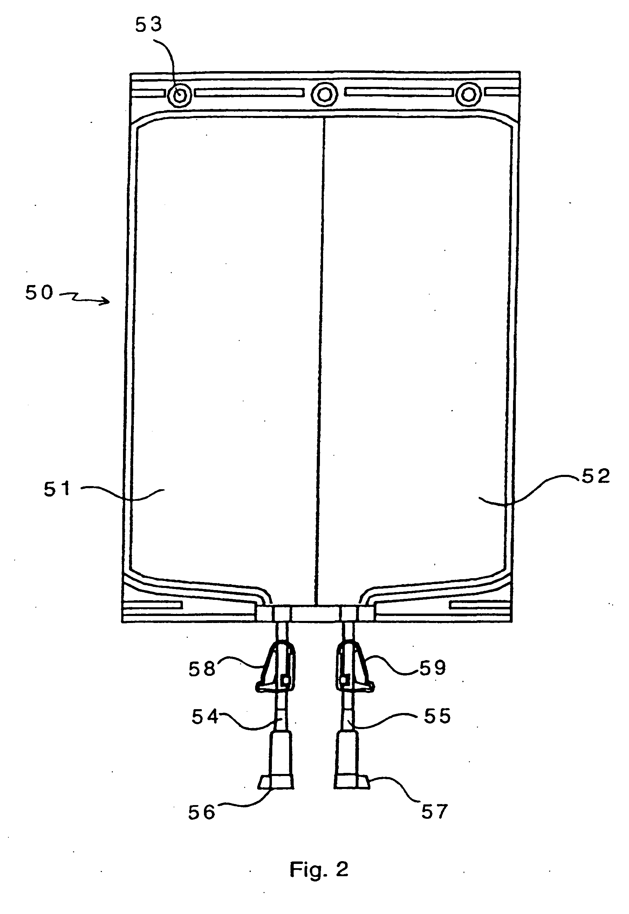

Le système d'hémodialyse décrit plus haut est équipé d'une poche 50 de matière plastique souple transparente, telle que représentée sur la figure 2, comprenant deux compartiments 51 et 52, correspondant respectivement au conteneurs 18 et 21 de la figure 1. La poche 50 est munie à sa partie supérieure d'oeillets 53 permettant de la suspendre en position verticale à un support approprié. Chaque compartiment 51, 52 est équipé à sa base d'une tubulure d'accès 54, 55 munie à son extrémité d'un élément de connexion 56, 57 destiné à coopérer avec un élément de connexion complémentaire fixé à l'extrémité des canalisations secondaires 16, 17 du dispositif 14 de préparation de liquide de dialyse. Un clamp 58, 59 est disposé sur chacune des tubulure 54, 55.

Le compartiment 51 (conteneur 18) contient les substances suivantes, dans les concentrations suivantes:
NaCl : 284,31 g/l
KCl : 19,57 g/l
CaCl2 : 10,29 g/l
MgCl2 : 2,63 g/l
glucose anhydre : 35 g/l

Le compartiment 52 (conteneur 21) contient les substances suivantes, dans les concentrations suivantes:
NaCl : 284,31 g/l
KCl : 0 g/l
CaCl2 : 10,29 g/l
MgCl2 : 2,63 g/l
glucose anhydre : 35 g/l

Au moyen de ces deux solutions, il est possible, selon l'invention, de préparer un liquide de dialyse ayant une concentration en sodium comprise entre environ 130 mEq/l et environ 155 mEq/l, et une concentration en potassium variant au cours d'une séance de traitement entre une valeur initiale comprise entre environ 2,5 mEq/l et environ 5,5 mEq/l, et une valeur finale comprise entre environ 1 mEq/l et environ 2mEq/l.

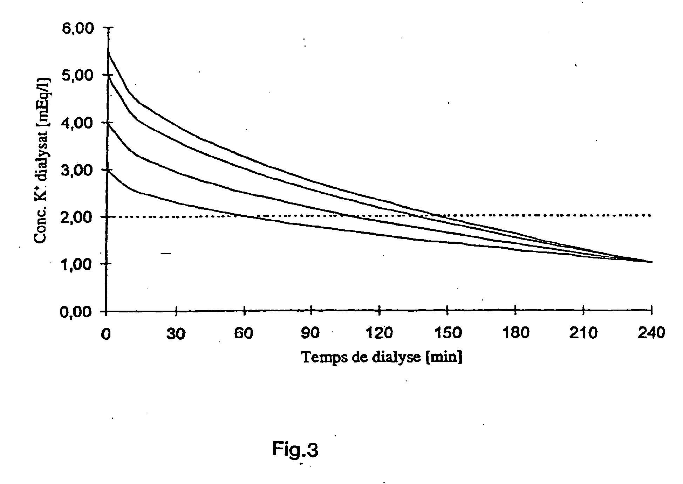

La figure 3 représente quatre profils de variation de la concentration en potassium d'un liquide de dialyse qui peuvent être réalisés au moyen du dispositif de préparation de liquide de dialyse 14 connecté de la poche 50 à deux compartiments contenant les solutions concentrées qui viennent d'être décrites. Sur cette figure, on a représenté en traits interrompus la concentration constante en potassium d'un liquide de dialyse classique, soit 2 mEq/l.

L'appareil d'hémodialyse qui vient d'être décrit fonctionne de la façon suivante.

Un opérateur communique à l'unité de commande 30, par l'intermédiaire du clavier 32, des valeurs de consignes classiques correspondant aux divers paramètres du traitement (prescription), à savoir, le débit du sang Qb, le débit du liquide de dialyse Qd, le débit de perfusion Qinf de la solution de bicarbonate, la perte de poids totale WL (quantité d'eau plasmatique à retirer du patient par ultrafiltration), la durée totale T de la séance, et la concentration en sodium du liquide de dialyse.

Conformément à l'invention, l'opérateur communique aussi à l'unité de commande une information, ou une série d'informations concernant la concentration en potassium du liquide de dialyse, qui peut être soit une valeur de consigne fixe, soit l'un des profils de variation préalablement mis en mémoire dans l'unité de commande, correspondant par exemple à l'un des graphes de la figure 3. L'opérateur peut aussi créer et mettre en mémoire un profil approprié à un client particulier.

Selon une variante de l'invention, la concentration en potassium du liquide de dialyse est ajustée par l'unité de commande 30 de la façon suivante : un liquide de dialyse ayant une concentration en potassium correspondant à une valeur de consigne prédéterminée est mis initialement en circulation dans l'hémodialyseur 1, et cette valeur de consigne est comparée à la valeur de la concentration du potassium dans le liquide usé, mesurée par la sonde 26. L'unité de commande 30 commande ultérieurement les pompes 19, 22 du dispositif 14 de préparation de liquide de traitement de façon que la différence entre la valeur de consigne et la valeur mesurée reste sensiblement égale à une valeur donnée, correspondant à une différence acceptable pour le patient entre la concentration en potassium du plasma et celle du liquide de dialyse.

Après qu'un kit de solutions concentrées, tel que la poche décrite plus haut, a été connecté aux canalisations 16, 17 du dispositif de préparation de liquide de dialyse 14, le circuit de liquide de dialyse est rempli de liquide de dialyse. Pour ce faire, la canalisation principale 15 est raccordée à une source d'eau courante et les pompes 19, 22, 25, 27 sont mises en fonctionnement. Les pompes 19 et 22 sont réglées par l'unité de commande 30 de façon que la concentration en potassium et la concentration en sodium du liquide de dialyse soient égales aux valeurs de consigne correspondantes. Les pompes de circulation de liquide de dialyse 25, 27 sont réglées par l'unité de commande 30 de façon que le débit de la pompe 25 située en amont de l'hémodialyseur 1 soit égal au débit de consigne Qd (500 ml/min, par exemple) et que le débit de la pompe 27 située en aval de l'hémodialyseur 1 soit tel que les débits mesurés par les débitmètres 24, 28 soient égaux.

Simultanément au remplissage du circuit de liquide de dialyse avec le liquide de dialyse conforme à la prescription, le circuit de circulation extracorporelle de sang est rincé et rempli de liquide physiologique stérile.

Lorsque l'amorçage du circuit de liquide de dialyse et du circuit sang est achevé, le circuit sang est connecté au patient et le traitement proprement dit peut commencer : les pompes 19, 22 du dispositif 14 de préparation de liquide de dialyse, ainsi que les pompes de circulation 25, 27 du liquide de dialyse continuent de fonctionner, tandis que la pompe à sang 6, la pompe d'extraction 29 et la pompe de perfusion 10 sont mises en fonctionnement. La pompe à sang 6 est réglée au débit de consigne Qb, (200 ml/mn, par exemple), la pompe de perfusion 10 au débit de consigne Qinf, et la pompe d'extraction 29 est réglée à un débit QUF calculé par l'unité de commande 30 à partir des valeurs de consigne de la perte de poids totale WL, du débit de perfusion Qinf et de la durée totale du traitement T.

L'invention qui vient d'être décrite est susceptible de variantes.

Avec le dispositif de préparation selon l'invention, il est possible d'ajuster simultanément la concentration en potassium d'un liquide de dialyse selon un premier profil de variation déterminé et la concentration en sodium du même liquide de dialyse selon un deuxième profil de variation déterminé.

Une sonde de mesure de la concentration en potassium peut être montée sur la canalisation d'alimentation 12 pour fournir une valeur mesurée de la concentration en potassium qui sera utilisée, par exemple, pour calculer la différence entre cette valeur et la valeur mesurée en aval de l'hémodialyseur 1 par la sonde 26.

## Revendications

1. Système de traitement extracorporel de sang comprenant:
• Un Dispositif pour préparer un liquide de traitement sans bicarbonate à partir d'un liquide, tel que de l'eau, et de deux solutions concentrées comprenant:
- une canalisation (15) ayant une première extrémité destinée à être raccordée à une source de liquide tel que de l'eau et une seconde extrémité pour délivrer un liquide de traitement;
- une première solution concentrée contenant au moins du sodium, du calcium, du magnesium et du potassium;
- des premiers moyens d'injection (19) pour injecter dans la canalisation (15), à un débit Q1, la première solution;
- une deuxième solution concentrée contenant au moins du sodium et du potassium, le sodium ayant, dans la deuxième solution concentrée, la même concentration que dans la première solution concentrée, et le potassium ayant des concentrations différentes dans la première solution concentrée et la deuxième solution concentrée ;
- des seconds moyens d'injection (22) pour injecter dans la canalisation (15), à un débit Q2, la deuxième solution ;
• Une canalisation (12) d'alimentation en liquide de traitement pour relier la canalisation (15) du dispositif de traitement à une entrée d'un échangeur à membrane;
• Une canalisation (13) d'évacuation de liquide usé destinée à être connectée à une sortie de l'échangeur à membrane (1);
**caractérisé en ce qu'**il comprend en outre des moyens pour perfuser à un patient une troisième solution contenant au moins du bicarbonate, **en ce que** la deuxième solution concentrée contient en outre du calcium et du magnésium, dans des concentrations respectivement égales aux concentrations du calcium et du magnésium dans la première solution concentrée, et **en ce qu'**il comporte en outre des moyens (20, 23, 30) de réglage pour régler les premiers et les seconds moyens (19, 22) d'injection et ajuster le débit d'injection Q1 et le débit d'injection Q2 de la première et de la deuxième solutions concentrées de façon que, à tout moment, la solution diluée résultant du mélange du liquide et des solutions concentrées ait une concentration souhaitée en sodium et une concentration souhaitée en potassium indépendantes l'une de l'autre, les moyens (20, 23, 30) de réglage étant prévus pour faire varier au cours du temps le débit d'injection Q1 et le débit d'injection Q2 des solutions concentrées de façon que la concentration de potassium dans la solution diluée varie au cours du temps selon un profil prédéterminé et que la concentration de sodium dans la solution diluée varie au cours du temps selon un profil prédéterminé indépendamment de la concentration en potassium.

2. Systeme de traitement selon la revendication 1, **caractérisé en ce que** la concentration de potassium dans la deuxième solution concentrée est égale à zéro.

3. Systeme de traitement selon une des revendications 1 et 2, **caractérisé en ce que** les moyens de réglage (20, 23, 30) sont prévus pour maintenir constante la somme des débits d'injection Q1 + Q2 des solutions concentrées, de sorte que, pour un débit Q0 du liquide constant dans la canalisation, la concentration de sodium dans la solution diluée reste sensiblement constante.

4. Systeme de traitement selon une des revendications 1 à 3, **caractérisé en ce que** le profil de concentration prédéterminé de potassium est un profil décroissant dont la valeur initiale est comprise entre environ 2,5 mEq/l et environ 5,5 mEq/l et la valeur finale est comprise entre environ 1 mEq/l et environ 2mEq/l.

5. Système de traitement extracorporel du sang selon une des revendications 1 à 4 comprenant :
un premier dispositif de mesure de la concentration de potassium dans le liquide de traitement disposé sur la canalisation de préparation; un second dispositif de mesure de la concentration de potassium dans le liquide usé disposé sur la canalisation d'évacuation.

6. Système de traitement selon la revendication 5, **caractérisé en ce que** les moyens (20, 23, 30) de réglage sont prévus pour régler les premiers et seconds moyens (19,22) d'injection à partir des informations fournies par le premier et le second dispositif de mesure de la concentration de potassium.

## Patentansprüche

1. Extrakorporales Blutbehandlungssystem umfassend:
- eine Vorrichtung zur Vorbereitung einer bikarbonatfreien Behandlungsflüssigkeit ausgehend von einer Flüssigkeit, wie z.B. Wasser, und zwei konzentrierten Lösungen umfassend:
- eine Leitung (15) mit einem ersten Ende zum Anschluss an eine Quelle von Flüssigkeit wie z.B. Wasser, und einem zweiten Ende zur Lieferung einer Behandlungsflüssigkeit;
- eine erste konzentrierte Lösung enthaltend mindestens Natrium, Kalzium, Magnesium und Kalium;
- erste Injektionsmittel (19) zum Injizieren der ersten Lösung in die Leitung (15) bei einer Flussrate Q1;
- eine zweite konzentrierte Lösung enthaltend mindestens Natrium und Kalium, wobei Natrium in der zweiten konzentrierten Lösung dieselbe Konzentration wie in der ersten konzentrierten Lösung aufweist, und wobei Kalium verschiedene Konzentrationen in der ersten konzentrierten Lösung und in der zweiten konzentrierten Lösung aufweist;
- zweite Injektionsmittel (22) zum Injizieren der zweiten Lösung in die Leitung (15) bei einer Flussrate Q2;
- eine Leitung (12) zur Zuführung von Behandlungsflüssigkeit zur Verbindung der Leitung (15) der Behandlungsvorrichtung mit einem Eingang eines Membranaustauschers;
- eine Leitung (13) zum Ablassen von gebrauchter Flüssigkeit, die zur Verbindung mit einem Ausgang des Membranaustauschers (1) vorgesehen ist;
**dadurch gekennzeichnet, dass** es weiter Mittel zur Perfusion eines Patienten mit einer dritten Lösung enthaltend mindestens Bikarbonat umfasst, und dadurch, dass die zweite konzentrierte Lösung weiter Kalzium und Magnesium enthält, mit Konzentrationen, die jeweils den Kalzium- und Magnesiumkonzentrationen in der ersten konzentrierten Lösung entsprechen, und dadurch, dass sie weiter Einstellungsmittel (20, 23, 30) zum Einstellen der ersten und zweiten Injektionsmittel (19, 22) und zum Einstellen der Injektionsflussrate Q1 und der Injektionsflussrate Q2 der ersten bzw. zweiten konzentrierten Lösung umfasst, so dass jederzeit die verdünnte Lösung aus dem Mischen der Flüssigkeit und der konzentrierten Lösungen eine gewünschte Natriumkonzentration und eine gewünschte Kaliumkonzentration aufweist, die unabhängig voneinander sind, wobei die Einstellungsmittel (20, 23, 30) zum Ändern im Lauf der Zeit der Injektionsflussrate Q1 und der Injektionsflussrate Q2 der konzentrierten Lösungen vorgesehen sind, so dass die Kaliumkonzentration sich in der verdünnten Lösung im Lauf der Zeit nach einem vorbestimmten Profil ändert und die Natriumkonzentration sich in der verdünnten Lösung im Lauf der Zeit nach einem vorbestimmten Profil unabhängig von der Kaliumkonzentration ändert.

2. Behandlungssystem nach Anspruch 1, **dadurch gekennzeichnet, dass** die Kaliumkonzentration in der zweiten konzentrierten Lösung gleich Null ist.

3. Behandlungssystem nach einem der Ansprüche 1 und 2, **dadurch gekennzeichnet, dass** die Einstellungsmittel (20, 23, 30) zum konstant Halten der Summe der Injektionsflussraten Q1 + Q2 der konzentrierten Lösungen vorgesehen sind, so dass bei einer konstanten Flussrate Q0 der Flüssigkeit in der Leitung die Natriumkonzentration in der verdünnten Lösung im Wesentlichen konstant bleibt.

4. Behandlungssystem nach einem der Ansprüche 1 bis 3, **dadurch gekennzeichnet, dass** das vorbestimmte Kalium-Konzentrationsprofil ein abnehmendes Profil ist, dessen Anfangswert ca. 2,5 mEq/l bis ca. 5,5 mEq/l beträgt und dessen Endwert ca. 1 mEq/l bis ca. 2 mEq/l beträgt.

5. Extrakorporales Blutbehandlungssystem nach einem der Ansprüche 1 bis 4 umfassend:
eine erste Messvorrichtung zur Messung der Kaliumkonzentration in der Behandlungsflüssigkeit, die an der Vorbereitungsleitung angeordnet ist;
eine zweite Messvorrichtung zur Messung der Kaliumkonzentration in der gebrauchten Flüssigkeit, die an der Ablassleitung angeordnet ist.

6. Behandlungssystem nach Anspruch 5, **dadurch gekennzeichnet, dass** die Einstellungsmittel (20, 23, 30) zum Einstellen der ersten und zweiten Injektionsmittel (19, 22) ausgehend von Informationen, die von der ersten und zweiten Messvorrichtung zur Messung der Kaliumkonzentration geliefert werden, vorgesehen sind.

## Claims

1. An extracorporeal blood treatment system comprising:
- a device for preparing a bicarbonate-free treatment liquid starting from a liquid, such as water, and two concentrated solutions comprising:
- a channel (15) having a first end designed to be hooked up to a source of liquid such as water, and a second end for delivering a treatment liquid;
- a first concentrated solution containing at least sodium, calcium, magnesium and potassium;
- first injection means (19) for injecting the first solution into the channel (15) at a flow rate Q1;
- a second concentrated solution containing at least sodium and potassium, the sodium having in the second concentrated solution the same concentration as in the first concentrated solution, and the potassium having different concentrations in the first concentrated solution and the second concentrated solution;
- second injection means (22) for injecting the second solution into the channel (15) at a flow rate Q2;
- a channel (12) for the supply of treatment liquid, for coupling the channel (15) of the treatment device to an inlet of a membrane exchanger;
- a channel (13) for the discharge of used liquid, designed to be connected to an outlet of the membrane exchanger (1);
**characterized in that** it further comprises means for perfusing a patient with a third solution containing at least bicarbonate, and **in that** the second concentrated solution further contains calcium and magnesium at concentrations that are the same as calcium and magnesium concentrations in the first concentrated solution, and **in that** it further comprises adjustment means (20, 23, 30) for adjusting the first and the second injection means (19, 22) and adjusting the injection flow rate Q1 and the injection flow rate Q2 of the first and of the second concentrated solutions, so that at any time the diluted solution obtained by mixing the liquid and the concentrated solutions has a desired sodium concentration and a desired potassium concentration that are independent from one another, the adjustment means (20, 23, 30) being designed for letting the injection flow rate Q1 and the injection flow rate Q2 of the concentrated solutions vary during time, so that potassium concentration in the diluted solution varies during time according to a predetermined profile and sodium concentration in the diluted solution varies during time according to a predetermined profile independently from potassium concentration.

2. The treatment system according to claim 1, **characterized in that** potassium concentration in the second concentrated solution is equal to zero.

3. The treatment system according to one of the claims 1 and 2, **characterized in that** the adjustment means (20, 23, 30) are designed to keep the sum of injection flow rates Q1 + Q2 of the concentrated solutions constant, so that for a constant flow rate Q0 of liquid in the channel, sodium concentration in the diluted solution remains basically constant.

4. The treatment device according to one of the claims 1 to 3, **characterized in that** the predetermined potassium concentration profile is a decreasing profile whose initial value is of about 2.5 mEq/l to about 5.5 mEq/l and whose final value is of about 1 mEq/l to about 2 mEq/l.

5. The extracorporeal blood treatment system according to one of the claims 1 to 4 comprising:
a first device for measuring potassium concentration in the treatment liquid, arranged on the preparation channel;
a second device for measuring potassium concentration in the used liquid, arranged on the discharge channel.

6. The treatment system according to claim 5, **characterized in that** the adjustment means (20, 23, 30) are designed to adjust the first and second injection means (19, 22) starting from information given by the first and the second device for measuring potassium concentration.
